(19) Europäisches Patentamt
European Patent Office
Office européen des brevets

(11) Veröffentlichungsnummer: **0 278 346 B1**

(12) # EUROPÄISCHE PATENTSCHRIFT

(45) Veröffentlichungstag der Patentschrift: **05.06.91 Patentblatt 91/23**

(51) Int. Cl.[5]: **C07D 249/08,** C07D 303/08, C07C 49/813, A61K 31/41, A01N 43/64

(21) Anmeldenummer: **88101367.6**

(22) Anmeldetag: **01.02.88**

(54) **1-(2,4-Difluorphenyl)-1-(1-fluorcyclopropyl)-2-(1,2,4-triazol-1-yl)-ethan-1-ol.**

Die Akte enthält technische Angaben, die nach dem Eingang der Anmeldung eingereicht wurden und die nicht in dieser Patentschrift enthalten sind.

(30) Priorität: **12.02.87 DE 3704261**

(43) Veröffentlichungstag der Anmeldung: **17.08.88 Patentblatt 88/33**

(45) Bekanntmachung des Hinweises auf die Patenterteilung: **05.06.91 Patentblatt 91/23**

(84) Benannte Vertragsstaaten: **BE CH DE FR GB IT LI NL**

(56) Entgegenhaltungen:
**DE-A- 3 440 116**
**DE-A- 3 535 456**

(73) Patentinhaber: **BAYER AG**
**W-5090 Leverkusen 1 Bayerwerk (DE)**

(72) Erfinder: **Böckmann, Klaus, Dr.**
**Andreas-Gryphius-Strasse 7**
**W-5000 Köln 80 (DE)**
Erfinder: **Stroech, Klaus, Dr.**
**Rolsbergerstrasse 2**
**W-5060 Solingen 19 (DE)**
Erfinder: **Dutzmann, Stefan, Dr.**
**Leinenweberweg 33**
**W-4000 Düsseldorf 13 (DE)**
Erfinder: **Reinecke, Paul, Dr.**
**Steinstrasse 8**
**W-5090 Leverkusen 3 (DE)**

**EP 0 278 346 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft das neue 1-(2,4-Difluorphenyl)-1-(1-fluorcyclopropyl)-2-(1,2,4-triazol-1-yl)-ethan-1-ol, ein Verfahren zu dessen Herstellung und dessen Verwendung zur Bekämpfung von Pseudocercosporella herpotrichoides.

Es ist bereits bekannt geworden, daß bestimmte Azolylmethyl-cyclopropyl-carbinol-Derivate eine gute fungizide Wirksamkeit besitzen (vgl. EP-A-0 180 136). Eine spezielle Verwendung dieser Stoffe gegen Pseudocercosporella herpotrichoides ist jedoch noch nicht beschrieben worden.

Weiterhin ist bereits bekannt, daß N-[2-(2,4,6-Trichlorphenoxy)-ethyl]-N-propyl-1H-imidazol-1-carboxamid zur Bekämpfung von Pseudocercosporella herpotrichoides geeignet ist (vgl. US-A-3 991 071 und US-A-4 080 462). Beiniedrigen Aufwandmengen ist die Wirksamkeit dieses Stoffes aber nicht immer befriedigend.

Es wurde nun das neue 1-(2,4-Difluorphenyl)-1-(2-fluorcyclopropyl)-2-(1,2,4-triazol-1-yl)-ethan-1-ol der Formel

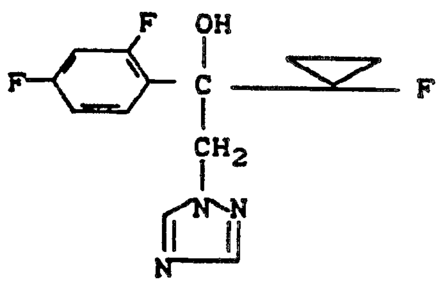

(I)

gefunden.

Weiterhin wurde gefunden, daß man das neue 1-(2,4-Difluorphenyl)-1-(1-fluorcyclopropyl)-2-(1,2,4-triazol-1-yl)-ethan-1-ol der Formel (I) erhält, wenn man

a) in einer ersten Stufe 2,4-Difluorphenyl-1-fluorcyclopropyl-keton der Formel

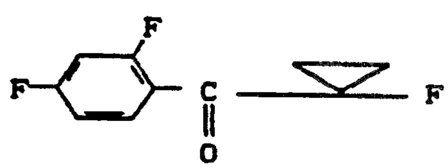

(II)

entweder

α) mit Dimethyloxosulfonium-methylid der Formel

$$(CH_3)_2\ \overset{\delta\oplus}{SO}\ \overset{\delta\ominus}{CH_2} \qquad (III)$$

oder

ß) mit Dimethylsulfonium-methylid der Formel

$$(CH_3)_2\ \overset{\delta\oplus}{S}\ \overset{\delta\ominus}{CH_2} \qquad (IV)$$

in Gegenwart eines Verdünnungsmittels umsetzt und

b) in einer zweiten Stufe das dabei entstehende 1-(2,4-Difluorphenyl)-1-(1-fluor-cyclopropyl)-oxiran der Formel

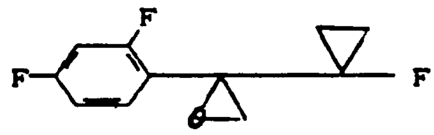

(V)

mit 1,2,4-Triazol der Formel

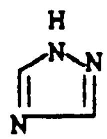

(VI)

in Gegenwart eines Säurebindemittels und in Gegenwart eines Verdünnungsmittels umsetzt.

Schließlich wurde gefunden, daß das neue 1-(2,4-Difluorphenyl)-1-(1-fluorcyclopropyl)-2-(1,2,4-triazol-1-yl)-ethan-1-ol der Formel (I) sehr gut zur Bekämpfung von Pseudocercosporella herpotrichoides geeignet ist.

Überraschenderweise zeigt der erfindungsgemäße Wirkstoff der Formel (I) beim Einsatz gegen Pseudocercosporella herpotrichoides eine wesentlich bessere Wirksamkeit als das N-[2-(2,4,6-Trichlorphenoxy)-ethyl]-N-propyl-1H-imidazol-carboxamid, welches ein anerkannt gut wirksamer und chemisch ähnlicher Wirkstoff gleicher Wirkungsrichtung ist.

Verwendet man bei der Durchführung des erfindungsgemäßen Verfahrens Dimethyloxosulfonium -methylid als Methylengenerator, so läßt sich dessen Verlauf durch das folgende Formelschema veranschaulichen :

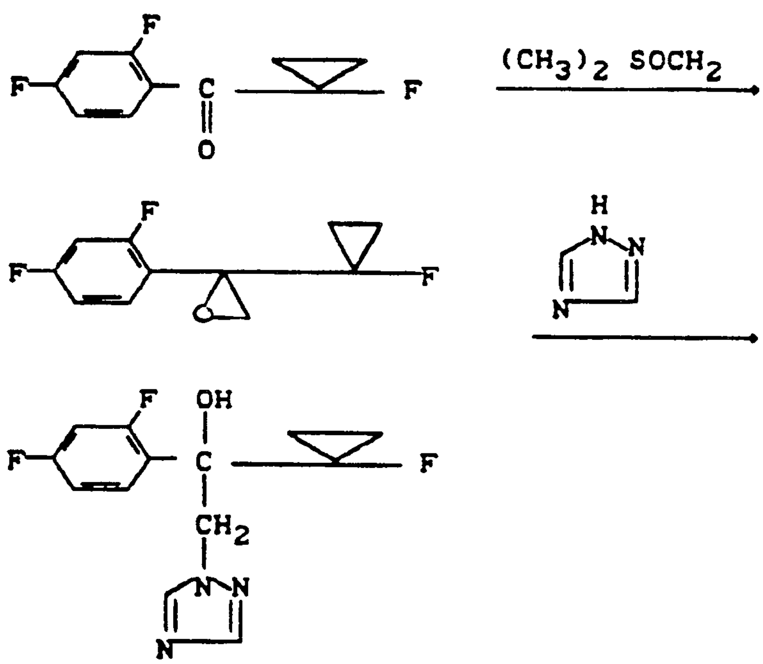


Das für das erfindungsgemäße Verfahren als Ausgangsstoff benötigte 2,4-Difluorphenyl-1-fluorcyclopropyl-keton der Formel (II) ist bisher noch nicht bekannt. Es läßt sich herstellen, indem man 2,4-Difluorphenyl-(3-chlor-1-fluor-propyl)-keton der Formel

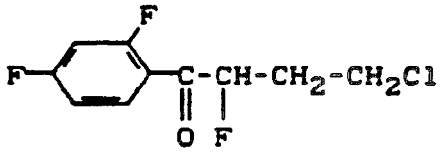

(VII)

in Gegenwart eines Säurebindemittels und in Gegenwart eines Verdünnungsmittels umsetzt.

Das bei dem obigen Verfahren als Ausgangsstoff benötigte 2,4-Difluorphenyl-(3-chlor-1-fluor-propyl)-keton der Formel (VII) läßt sich herstellen, indem man 2,4-Difluorphenyl-(1-brom-3-chlor-propyl)-keton der Formel

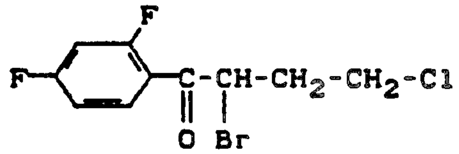

(VIII)

mit Natriumfluorid oder Kaliumfluorid in Gegenwart eines inerten organischen Verdünnungsmittels, wie zum Beispiel Benzol, und in Gegenwart eines Komplexbildners, wie zum Beispiel 18-Krone-6, bei Temperaturen zwischen 20 und 150°C umsetzt.

Als Säurebindemittel kommen bei dem obigen Verfahren zur Herstellung der Verbindung der Formel (II) alle üblichen anorganischen und organischen Basen in Betracht. Vorzugsweise verwendbar sind Alkalimetallcarbonate, wie Natrium- und Kaliumcarbonat, Alkalimetallhydroxide, wie Natrium- und Kaliumhydroxid, Alkalimetallalkoholate, wie Natrium- und Kaliummethylat, -ethylat und -tert.-butylat; Alkalimetallhydride, wie Natriumhydrid, sowie niedere tertiäre Alkylamine, Cycloalkylamine und Aralkylamine, wie insbesondere Triethylamin.

Als Verdünnungsmittel können bei dem obigen Verfahren zur Herstellung der Verbindung der Formel (II) alle unter den Reaktionsbedingunen inerten organischen Solventien eingesetzt werden. Vorzugsweise verwendbar sind Alkohole, wie Methanol, Ethanol, Methoxyethanol, Propanol oder tert.-Butanol, ferner Ketone, wie Aceton und 2-Butanon, außerdem Nitrile, wie Acetonitril, weiterhin Ester, wie Essigester, darüber hinaus Ether, wie Dioxan, aromatische Kohlenwasserstoffe, wie Benzol oder Toluol, und auch Amide, wie Dimethylformamid.

Die Reaktionstemperaturen können bei der Durchführung des obigen Verfahrens zur Herstellung der Verbindung der Formel (II) in einem größeren Bereich variiert werden. Im allgemeinen arbeitet man bei Temperaturen zwischen 0°C und 200°C, vorzugsweise zwischen 20°C und 150°C.

Bei der Herstellung der Verbindung der Formel (II) nach dem obigen Verfahren setzt man auf 1 Mol an 2,4-Difluorphenyl-(3-chlor-1-fluor-propyl)-keton der Formel (VII) vorzugsweise 1 bis 2 Mol an Base ein. Die Isolierung der Verbindung der Formel (II) erfolgt in üblicher Weise.

Das bei dem erfindungsgemäßen Verfahren als Reaktionskomponente benötigte Dimethyloxosulfoniummethylid der Formel (III) ist bekannt (vgl. J. Am. Chem. Soc. 87, 1363-1364 (1965). Es wird bei der obigen Umsetzung in frisch zubereitetem Zustand verarbeitet, indem man es in situ durch Umsetzung von Trimethyloxosulfoniumjodid mit Natriumhydrid oder Natriumamid, insbesondere mit Kalium-tert.-butylat oder Natriummethylat, in Gegenwart eines Verdünnungsmittels erzeugt.

Das bei dem erfindungsgemäßen Verfahren außerdem als Reaktionskomponente in Betracht kommende Dimethylsulfonium-methylid der Formel (IV) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfoniummethylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Das bei dem erfindungsgemäßen Verfahren als Zwischenprodukt auftretende 1-(2,4-Difluorphenyl)-1-(1-fluorcyclopropyl)-oxiran der Formel (V) ist noch nicht bekannt.

Als Verdünnungsmittel kommen bei der Durchführung der ersten Stufe des erfindungsgemäßen Verfahrens inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung der ersten Stufe des erfindungsgemäßen Verfahrens in einem größeren Bereich variiert werden. Im allgemeinen arbeitet man zwischen 0 und 100°C, vorzugsweise zwischen 10 und 60°C.

Bei der Durchführung der ersten Stufe des erfindungsgemäßen Verfahrens setzt man auf 1 Mol 2,4-Difluorphenyl-1-fluorcyclopropyl-keton der Formel (II) vorzugsweise 1 bis 3 Mol an Dimethyloxosulfonium-methylid der Formel (III) bzw. an Dimethylsulfonium-methylid der Formel (IV) ein. Die Isolierung des Zwischenproduktes der Formel (V) erfolgt nach üblichen Methoden.

Die zweite Stufe des erfindungsgemäßen Verfahrens wird in Gegenwart einer Base durchgeführt. Dabei kommen alle üblicherweise verwendbaren anorganischen und organischen Basen in Betracht. Vorzugsweise verwendbar sind Alkalicarbonate, wie z.B. Natrium- und Kaliumcarbonat ; Alkalihydroxide, wie z.B. Natriumhydroxid ; Alkalialkoholate, wie z.B. Natrium- und Kaliummethylat und – ethylat ; Alkalihydride, wie z.B. Natriumhydrid, sowie niedere tertiäre Alkylamine, Cycloalkylamine und Aralkylamine, wie insbesondere Triethylamin.

Als Verdünnungsmittels kommen für die zweite Stufe des erfindungsgemäßen Verfahrens inerte organische Lösungsmittel in Frage. Vorzugsweise verwendbar sind Nitrile, wie insbesondere Acetonitril ; aromatische Kohlenwasserstoffe, wie Benzol, Toluol und Dichlorbenzol ; Formamide, wie insbesondere Dimethylformamid, sowie Hexamethylphosphorsäuretriamid.

Die Reaktionstemperaturen können bei der Durchführung der zweiten Stufe des erfindungsgemäßen Verfahrens in einem größeren Bereich variiert werden. Im allgemeinen arbeitet man bei Temperaturen zwischen 0°C und 200°C, vorzugsweise zwischen 60°C und 150°C.

Bei der Durchführung der zweiten Stufe des erfindungsgemäßen Verfahrens setzt man vorzugsweise auf 1 Mol an Oxiran der Formel (V), 1 bis 2 Mol an 1,2,4-Triazol und 1 bis 2 Mol an Base ein. Die Isolierung des Endproduktes erfolgt in üblicher Weise.

Der erfindungsgemäße Wirkstoff eignet sich hervorragend zur Bekämpfung von Pseudocercosporella herpotrichoides, den Erreger der Halmbruchkrankheit bei Getreide. Bevorzugt ist der Einsatz des erfindungsgemäßen Wirkstoffes bei der Bekämpfung von Pseudocercosporella herpotrichoides bei Weizen und Gerste.

Der Wirkstoff kann in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche beinormaler Temperatur und unter Normaldruck gasförmig sind, z.B. Aerosol-Treibgase, wie Halogenkohlenwasserstoffe sowie Butan, Propan, Stickstoff und Kohlendioxid ;
als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gewichtsprozent Wirkstoff, vorzugsweise

zwischen 0,5 und 90%.

Der erfindungsgemäße Wirkstoff kann in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Der Wirkstoff kann als solcher, in Form seiner Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung gechieht in üblicher Weise, z. B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Sattgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02% am Wirkungsort erforderlich.

Die Herstellung und die Verwendung des erfindungsgemäßen Wirkstoffes geht aus den folgenden Beispielen hervor.

Herstellungsbeispiel

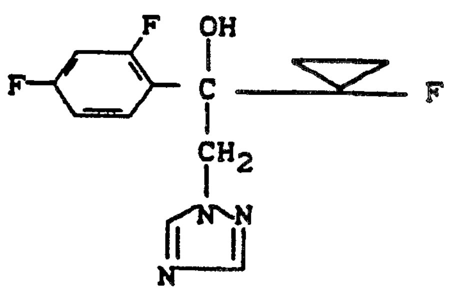

(I)

1. Stufe

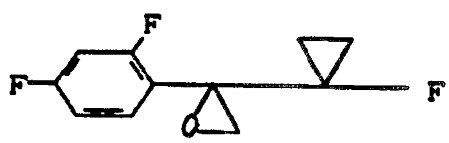

(V)

In eine Mischung aus 2,3 g (77 mMol) Natriumhydrid (80%ig) und 16,2 g (74 mMol) Trimethyloxosulfoniumiodid werden bei 10°C unter Stickstoffatmosphäre 60 ml absolutes Dimethylsulfoxid getropft. Man rührt 1 Stunde bei Raumtemperatur nach und tropft dann eine Lösung von 13 g (65 mMol) 2,4-Difluorphenyl-1-fluorcyclopropylketon in 20 ml absolutem Dimethylsulfoxid zu. Das Gemisch wird 2 Tage bei 20°C stehengelassen, dann 1 Stunde auf 40°C erwärmt und in Wasser gegossen. Das entstehende Gemisch wird mehrfach mit Essigester extrahiert, die vereinigten organischen Phasen werden über Natriumsulfat getrocknet und durch Abziehen des Lösungsmittels unter vermindertem Druck eingeengt. Man erhält auf diese Weise 13 g (94% der Theorie) an 1-(2,4-Difluorphenyl)-1-(1-fluorcyclopropyl)-oxiran.
$^1$H-NMR (60 MHz, $CDCl_3$) :

δ = 0,7-1,5 (m, 4H), 2,8-3,2 (m, 2H),
6,5-7,1 (m, 2H), 7,2-7,6 (m, 1H).

2. Stufe

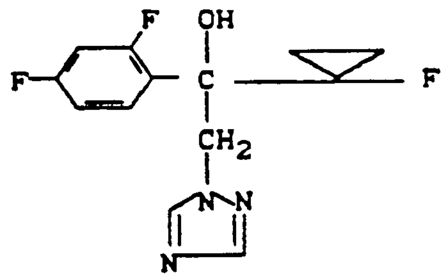

(I)

In ein Gemisch aus 14 g (200 mMol) 1,2,4-Triazol und 1,7 g (15 mMol) Kalium-tert.-butylat in 50 ml absolutem Dimethylformamid wird bei 80°C unter Rühren eine Lösung von 13 g (61 mMol) 1-(2,4-Difluorphenyl)-1-(1-fluorcyclopropyl)-oxiran in 20 ml absolutem Dimethylformamid getropft. Man erhitzt weitere 8 Stunden unter Rühren auf 80°C und zieht dann das Lösungsmittel unter vermindertem Druck ab. Der verbleibende Rückstand wird in Essigester aufgenommen. Man wäscht die entstehende Lösung mit Wasser, trocknet über Natriumsulfat und engt durch Abziehen des Lösungsmittels unter vermindertem Druck ein. Das verbleibende Produkt wird an einer Kieselgel-Säule chromatographisch gereinigt (Laufmittel : Chloroform). Nach dem Eindampfen des Eluates erhält man 9 g (52% der Theorie) an 1-(2,4-Difluorphenyl)-1-(1-fluorcyclopropyl)-2-(1,2,4-triazol)-1-yl)-ethan-1-ol, das nach Umkristallisation aus Cyclohexan einen Schmelzpunkt von 112°C aufweist.

Herstellung von Vorprodukten :

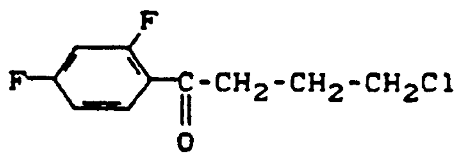

In eine Mischung aus 200 g (1,75 Mol) 1,3-Difluorbenzol und 256 g (1,92 Mol) wasserfreiem Aluminiumchlorid werden bei Raumtemperatur unter Rühren 255 g (1,81 Mol) 4-Chlorbuttersäurechlorid getropft. Das Gemisch wird 4 Stunden bei 30°C gerührt und dann auf 1200 g Eis gegossen. Man gibt 1200 ml Methylenchlorid hinzu, trennt die organische Phase ab, extrahiert die wäßrige Phase mit Methylenchlorid, wäscht die vereinigten organischen Phasen mit Wasser, trocknet über Natriumsulfat und engt durch Abziehen des Lösungsmittels unter vermindertem Druck ein. Der verbleibende Rückstand wird destilliert. Man erhält auf diese Weise 326 g (85% der Theorie) an 2,4-Difluorphenyl-3-chlorpropyl-keton.
Siedepunkt : 92°C/0,2 mbar

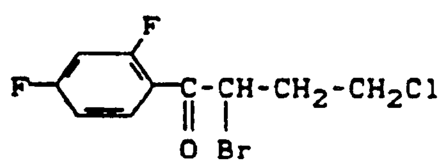

(VIII)

In eine Lösung von 555 g (2,54 Mol) 2,4-Difluorphenyl-3-chlorpropyl-keton in 1100 ml Methylenchlorid wird bei Raumtemperatur unter Rühren eine Lösung von 410,7 g (2,57 Mol) Brom in 400 ml Methylenchlorid eingetropft.

Es wird 1 Stunde bei Raumtemperatur nachgerührt. Danach wird das Lösungsmittel unter vermindertem Druck abgezogen. Man erhält auf diese Weise 715 g (95% der Theorie) an 2,4-Difluorphenyl-(1-brom-3-chlorpropyl)-keton.
$^1$H-NMR (60 MHz, $CDCl_3$) :

δ = 2,4-2,85 (m, 2H), 3,5-4,0 (m, 2H),
5,5 (dd, 1H), 6,8-7,3 (m, 2H), 7,9-8,3 (m, 1H).

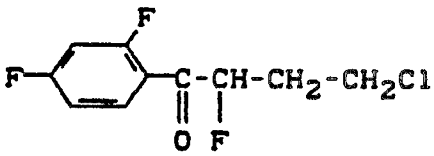

(VII)

Zu 61 g an der Ölpumpe bei 150°C getrocknetem Kaliumfluorid werden 450 ml absolutes Benzol und 24,5 g 18-Krone-6 gegeben. Dazu fügt man 150 g (0,5 Mol) 2,4-Difluorphenyl-(1-brom-3-chlorpropyl)-keton und erhitzt 8 Stunden unter Rückfluß. Nach dem Abkühlen setzt man 250 ml Essigester zu, wäscht die organische Phase dreimal mit Wasser, trocknet über Natriumsulfat und destilliert das Lösungsmittel ab. Man erhält 135 g 2,4-Difluorphenyl-(3-chlor-1-fluorpropyl)-keton, das ohne zusätzliche Reinigung weiter umgesetzt wird.

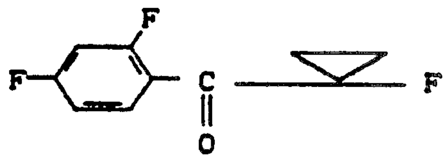

(II)

In ein Gemisch aus 85 g (0,75 Mol) Kalium-tert.-butylat und 240 ml tert.-Butanol werden bei 40°C unter Rühren 135 g 2,4-Difluorphenyl-(3-chlor-1-fluor-propyl)-keton eingetropft. Das Reaktionsgemisch wird 2 Stunden bei 40°C gerührt und dann auf 500 g Eis gegossen. Man extrahiert das entstehende Gemisch dreimal mit je 250 ml Essigester, wäscht die vereinigten organischen Phasen zweimal mit Wasser, trocknet über Natriumsulfat und engt durch Abziehen des Lösungsmittels unter vermindertem Druck ein. Nach Destillation des verbleibenden Rückstandes erhält man 13,3 g (13,3% der Theorie; bezogen auf 2,4-Difluorphenyl-(1-brom-3-chlor-propyl)-keton) an 2,4-Difluorphenyl-1-fluorcyclopropyl-keton.
Siedepunkt: 78°C/0,2 mbar

Beispiel A

Pseudocercosporella-Test (Getreide)/Sproßbehandlung Feldversuch

| | |
|---|---|
| Getreideart: | Winterweizen |
| Parzellengröße: | 1 $m^2$ |
| Anzahl der Wiederholungen: | 3 |
| Befall durch: | Pseudocercosporella herpotrichoides |

Die Anwendung der Wirkstoffe erfolgt in handelsüblichen Formulierungen beim Schossen des Getreides.

Die Auswertung wird zu dem Zeitpunkt, bei dem die Krankheitssymptome vollständig und gut zu erkennen sind, durchgeführt.

Wirkstoffe, Wirkstoffkonzentrationen und Versuchsergebnisse gehen aus der folgenden Tabelle hervor.

**Tabelle A**

Pseudocercosporella-Test (Getreide) / Sproßbehandlung Feldversuch

| Wirkstoff | Wirkstoffaufwandmenge in g / ha | Krankheitsbefall in % der unbehandelten Kontrolle |
|---|---|---|
| bekannt: | | |
| 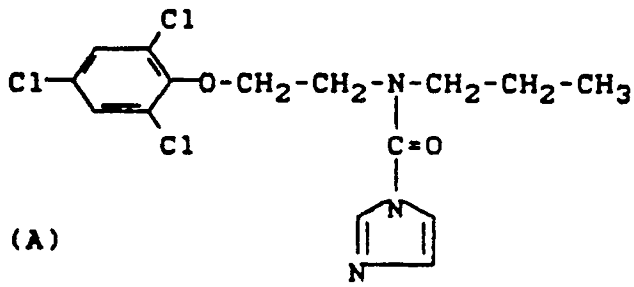 (A) | 250 | 84,1 |
| erfindungsgemäß: | | |
| 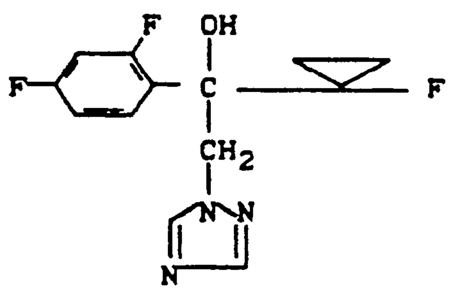 | 250 | 38,8 |

## Ansprüche

1. 1-(2,4-Difluorphenyl)-1-(1-fluorcyclopropyl)-2-(1,2,4-triazol-1-yl)-ethan-1-ol der Formel

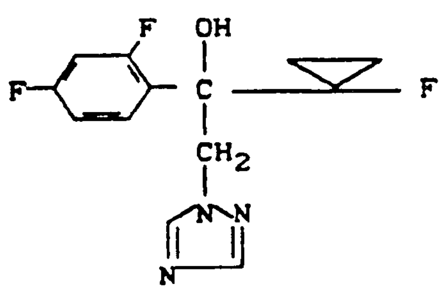

(I)

2. Verfahren zur Herstellung von 1-(2,4-Difluorphenyl)-1-(1-fluorcyclopropyl)-2-(1,2,4-triazol-1-yl)-ethan-1-ol der Formel

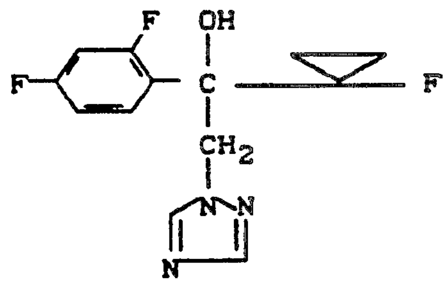

(I)

dadurch gekennzeichnet, daß man

a) in einer ersten Stufe 2,4-Difluorphenyl-1-fluorcyclopropyl-keton der Formel

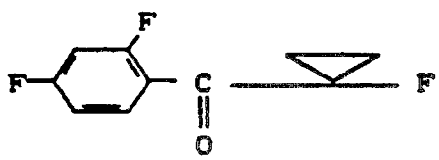

(II)

entweder

α) mit Dimethyloxosulfonium-methylid der Formel

$$\overset{\delta\oplus}{(CH_3)_2\,SO}\ \overset{\delta\ominus}{CH_2} \qquad (III)$$

oder

ß) mit Dimethylsulfonium-methylid der Formel

$$\overset{\delta\oplus}{(CH_3)_2\,S}\ \overset{\delta\ominus}{CH_2} \qquad (IV)$$

in Gegenwart eines Verdünnungsmittels umsetzt und

b) in einer zweiten Stufe das dabei entstehende 1-(2,4-Difluorphenyl)-1-(1-fluor-cyclopropyl)-oxiran der Formel

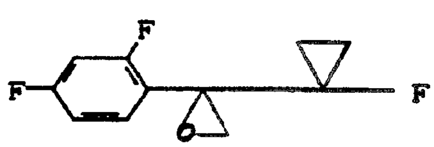

(V)

mit 1,2,4-Triazol der Formel

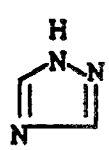

(VI)

in Gegenwart eines Säurebindemittels und in Gegenwart eines Verdünnungsmittels umsetzt.

3. Mittel zur Bekämpfung von Pseudocercosporella herpotrichoides, gekennzeichnet durch einen Gehalt an 1-(2,4-Difluorphenyl)-1-(1-fluorcyclopropyl)-2-(1,2,4-triazol-1-yl)-ethan-1-ol der Formel (I) gemäß Anspruch 1.

4. Verwendung von 1-(2,4-Difluorphenyl)-1-(1-fluorcyclopropyl)-2-(1,2,4-triazol)-1-yl)-ethan-1-ol der Formel (I) gemäß Anspruch 1 zur Bekämpfung von Pseudocercosporella herpotrichoides.

5. Verfahren zur Bekämpfung von Pseudocercosporella herpotrichoides, dadurch gekennzeichnet, daß man 1-(2,4-Difluorphenyl)-1-(1-fluorcyclopropyl)-2-(1,2,4-triazol-1-yl)-ethan-1-ol der Formel (I) gemäß Anspruch 1 auf derartige Pilze und/oder deren Lebensraum ausbringt.

6. Verfahren zur Herstellung von Mitteln zur Bekämpfung von Pseudocercosporella herpotrichoides, dadurch gekennzeichnet, daß man 1-(2,4-Difluorphenyl)-1-(1-fluorcyclopropyl)-2-(1,2,4-triazol-1-yl)-ethan-1-ol der Formel (I) gemäß Anspruch 1 mit Streckmitteln und/oder oberflächenaktiven Stoffen vermischt.

7. 2,4-Difluorphenyl-1-fluorcyclopropyl-keton der Formel

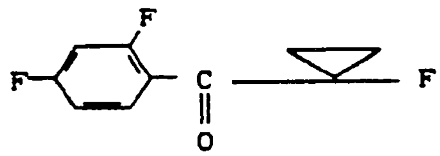

(II)

8. Verfahren zur Herstellung von 2,4-Difluorphenyl-1-fluorcyclopropyl-keton der Formel

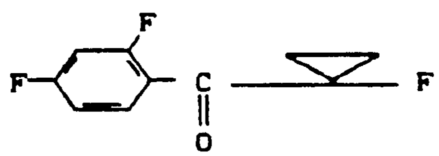

(II)

dadurch gekennzeichnet, daß man 2,4-Difluorphenyl-(3-chlor-1-fluor-propyl)-keton der Formel

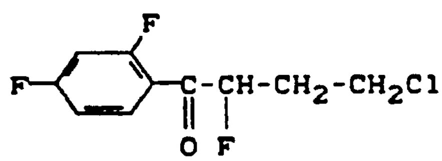

(VII)

in Gegenwart eines Säurebindemittels und in Gegenwart eines Verdünnungsmittels umsetzt.

9. 1-(2,4-Difluorphenyl)-1-(1-fluor-cyclopropyl)-oxiran der Formel

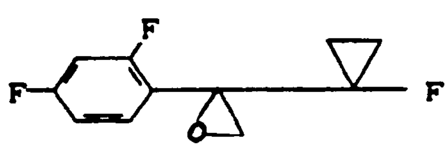

(V)

## Claims

1. 1-(2,4-Difluorophenyl)-1-(1-fluorocyclopropyl-2-(1,2,4-triazol-1-yl)-ethan-1-ol of the formula

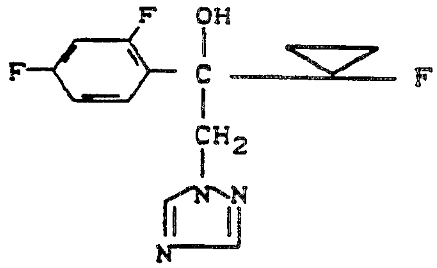

(I)

2. Process for the preparation of 1-(2,4-difluorophenyl)-1-(1-fluorocyclopropyl)-2-(1,2,4-triazol-1-yl)-ethan-1-ol of the formula

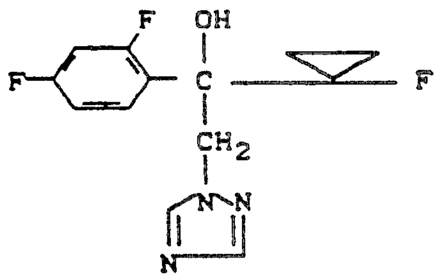

(I)

characterised in that

a) in a first stage, 2,4-difluorophenyl 1-fluorocyclopropyl ketone of the formula

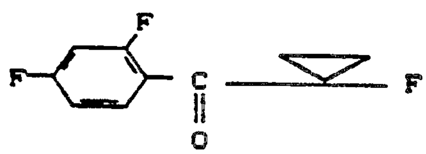

(II)

is reacted either

α) with dimethyloxosulphonium methylide of the formula

$$\overset{\delta^{\oplus}}{(CH_3)_2\,SO}\;\overset{\delta^{\ominus}}{CH_2} \qquad (III)$$

or

β) with dimethylsulphonium methylide of the formula

$$\overset{\delta^{\oplus}}{(CH_3)_2\,S}\;\overset{\delta^{\ominus}}{CH_2} \qquad (IV)$$

in the presence of a diluent and

b) in a second stage, the 1-(2,4-difluorophenyl)-1-(1-fluoro-cyclopropyl)-oxirane thereby formed, of the formula

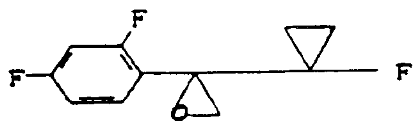

(V)

is reacted with 1,2,4-triazole of the formula

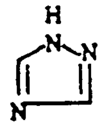

(VI)

in the presence of an acid-binding agent and in the presence of a diluent.

3. Agents for combating Pseudocercosporella herpotrichoides, characterised in that they contain 1-(2,4-difluorophenyl)-1-(1-fluorocyclopropyl)-2-(1,2,4-triazol-1-yl)-ethan-1-ol of the formula (I) according to Claim 1.

4. Use of 1-(2,4-difluorophenyl)-1-(1-fluorocyclopropyl)-2-(1,2,4-triazol-1-yl)-ethan-1-ol of the formula (I) according to Claim 1 for combating Pseudocercosporella herpotrichoides.

5. Method of combating Pseudocercosporella herpotrichoides, characterised in that 1-(2,4-difluorophenyl)-1-(1-fluorocyclopropyl)-2-(1,2,4-triazol-1-yl)-ethan-1-ol of the formula (I) according to Claim 1 is applied to such fungi and/or their environment.

6. Process for the preparation of agents for combating Pseudocercosporella herpotrichoides, characterised in that 1-(2,4-difluorophenyl)-1-(1-fluorocyclopropyl)-2-(1,2,4-triazol-1-yl)-ethan-1-ol of the formula (I) according to Claim 1 is mixed with extenders and/or surface-active substances.

7. 2,4-Difluorophenyl 1-fluorocyclopropyl ketone of the formula

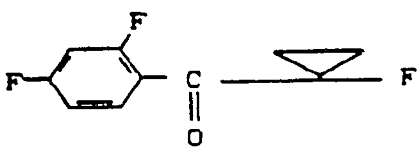

(II)

8. Process for the preparation of 2,4-difluorophenyl 1-fluorocyclopropyl ketone of the formula

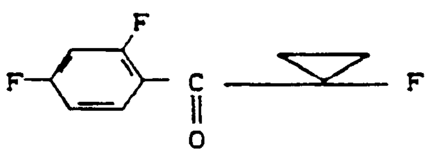

(II),

characterised in that 2,4-difluorophenyl 3-chloro-1-fluoro-propyl ketone of the formula

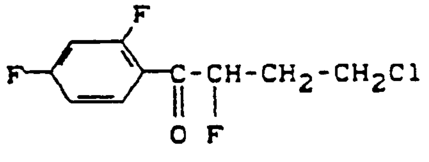

(VII)

is reacted in the presence of an acid-binding agent and in the presence of a diluent.

9. 1-(2,4-Difluorophenyl)-1-(1-fluoro-cyclopropyl)-oxirane of the formula

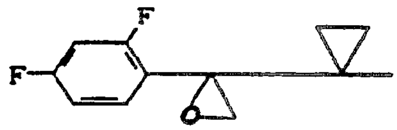 (V)

## Revendications

1. 1-(2,4-difluorophényl)-1-(1-fluorocyclopropyl)-2-(1,2,4-triazol-1-yl)-éthane-1-ol de formule

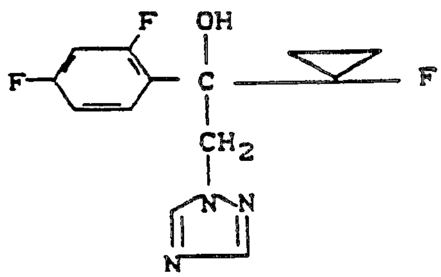 (I)

2. Procédé pour fabriquer le 1-(2,4-difluorophényl)-1-(1-fluorocyclopropyl)-2-(1,2,4-triazol-1-yl)-éthane-1-ol de formule

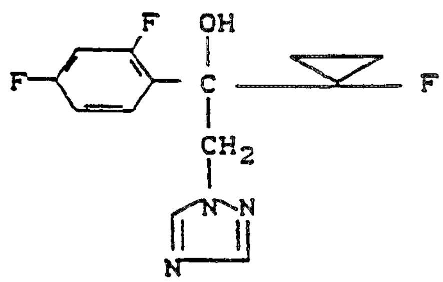 (I)

caractérisé en ce que :

a) dans une <u>première étape</u>, on fait réagir la 2,4-difluorophényl-1-fluorocyclopropyl-cétone de formule

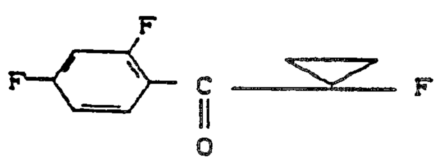 (II)

soit

α) avec du méthylure de diméthyloxosulfonium de formule

$$(CH_3)_2\ \overset{\delta\oplus}{SO}\ \overset{\delta\ominus}{CH_2} \qquad (III)$$

ou

β) avec du méthylure de diméthylsulfonium de formule

$$(CH_3)_2\overset{\delta^{\ominus}}{S}\ \overset{\delta^{\ominus}}{CH_2}$$ (IV)

en présence d'un agent de dilution, et

b) dans une seconde étape, on fait réagir le 1-(2,4-difluorophényl)-1-(1-fluorocyclopropyl)-oxiranne de formule

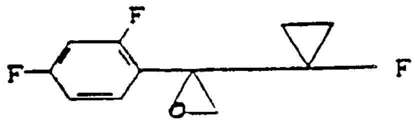 (V)

avec du 1,2,3-triazole de formule

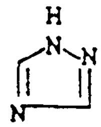 (VI)

en présence d'un fixateur d'acide et en présence d'un agent de dilution.

3. Produit pour la lutte contre Pseudocercosporella herpotrichoides, caractérisé en ce qu'il contient du 1-(2,4-difluorophényl)-1-(1-fluorocyclopropyl)-2-(1,2,4-triazol-1-yl)-éthane-1-ol de formule I selon la revendication 1.

4. Utilisation de 1-(2,4-difluorophényl)-1-(1-fluorocyclopropyl)-2-(1,2,4-triazol-1-yl)-éthane-1-ol de formule I selon la revendication 1 pour la lutte contre Pseudocercosporella herpotrichoides.

5. Procédé pour la lutte contre Pseudocercosporella herpotrichoides, caractérisé en ce que l'on applique le 1-(2,4-difluorophényl)-1-(1-fluorocyclopropyl)-2-(1,2,4-triazol-1-yl)-éthane-1-ol de formule I selon la revendication 1 sur ces champignons et/ou sur leur habitat.

6. Procédé pour la fabrication de produits pour la lutte contre Pseudocercosporella herpotrichoides, caractérisé en ce que l'on mélange le 1-(2,4-difluorophényl)-1-(1-fluorocyclopropyl)-2-(1,2,4-triazol-1-yl)-éthane-1-ol de formule I selon la revendication 1 avec des agents d'allongement et/ou des substances tensio-actives.

7. 2,4-difluorophényl-1-fluorocyclopropyl-cétone de formule

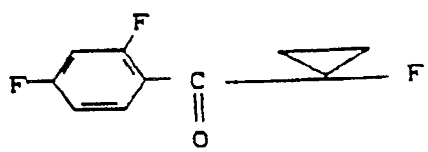 (II)

8. Procédé pour la fabrication de 2,4-difluorophényl-1-fluorocyclopropyl-cétone de formule

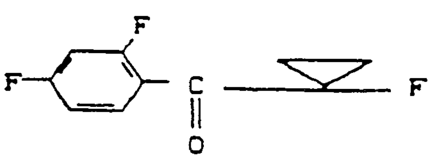 (II)

caractérisé en ce que l'on fait réagir la 2,4-difluorophényl-(3-chloro-1-fluoro-propyl)-cétone de formule

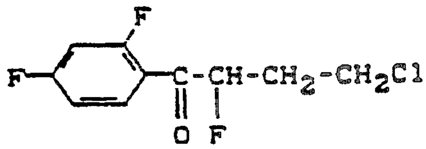

(VII)

en présence d'un fixateur d'acide et en présence d'un agent de dilution.

9. 1-(2,4-difluorophényl)-1-(1-fluorocyclopropyl)-oxiranne de formule

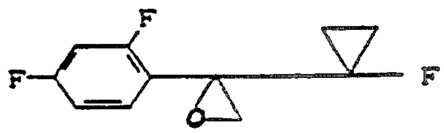

(V)